# EUROPEAN PATENT APPLICATION

(11) **EP 1 604 627 A1**
(43) Date of publication of application: **14.12.2005**
(21) Application number: 04721288.1
(22) Date of filing: 17.03.2004
(51) Int. Cl.: A61F 13/533

(54) **ABSORBENT ARTICLE HAVING DIVIDED ABSORBENT CORE**

(30) Priority: 17.03.2003 JP 2003071869
(71) Applicant: Koyo Corporation, Yokohama-shi, Kanagawa 247-0014 (JP)
(72) Inventor: TERADA, Sadayoshi, Kamakura-shi, Kanagawa 248-0025 (JP); YAMAZAKI, Rie, Hiratsuka-shi, Kanagawa 254-0903 (JP)
(74) Representative: Pingree, Oliver Norman
(86) International application number: PCT/JP2004/003526
(87) International publication number: WO 2004/082547

(57) **Abstract**

An absorbent article such as disposable diaper or sanitary napkin in which discharged liquid can be penetrated and diffused quickly. A rectangular shaped flat absorbent core interposed between a front surface sheet and a back surface sheet comprises at least one ditch traversing a point within a circle with a radius of 25 mm from its plane center to the periphery of the flat absorbent core so as to divide the flat absorbent core into not less than two parts.

## Description

### FIELD OF THE INVENTION

The present invention relates to an improved absorbent article such as a disposable diaper or a sanitary napkin, and more particularly to an absorbent article containing a flat absorbent core in which the absorbent core is divided into plural portions by means of at least one hollow gap (ditch), and sandwiched between a front surface sheet and a back surface sheet so as to maintain the configuration, which allows the absorbent core to perform its inherent liquid absorbency.

### BACKGROUND OF THE INVENTION

An absorbent article such as a disposable diaper or a sanitary napkin is basically composed of a flat absorbent core having excellent liquid absorbency which is sandwiched between a liquid permeable front sheet and a liquid impermeable back sheet. In most conventional absorbent articles, a flat absorbent core is adhered to both front and back surface sheets with a hot melt adhesive in order to prevent the core part from slipping and being displaced between the sheets.

However, such an adhesion of an absorbent core and both sheets using a hot melt adhesive is not preferable as it impairs an absorbency property of an absorbent core to some extent. Especially, when a hot melt adhesive is used to glue a front surface sheet and an absorbent core, there exists a hardened adhesive on the interface between the front surface sheet and the absorbent core, which reduces the liquid permeability of the front surface sheet and the absorbency of the absorbent core.

As an absorbent article in which a hot melt adhesive is not used on the interface between a flat absorbent core and a front and back surface sheet in which a flat absorbent core is not leaned or displaced between the front and the back surface sheet, the one in which an absorbent core is divided into plural portions, the periphery of each portion fixed firmly to the front and back surface sheet is known (See Reference 1). In this absorbent article, as all the periphery of the divided portions of the flat absorbent core is surrounded by the joint of the front surface sheet and the back surface sheet, deviation or slippage can be prevented without gluing the flat absorbent core and the above sheets with a hot melt adhesive. In addition to the above, there is known a conventional absorbent article having holes on a front surface side in which an absorbent core intervenes between a liquid pervious front sheet and a liquid impervious back sheet in the longitudinal direction of those sheets, in which a plurality of holes are arranged to extend towards the longitudinal direction while piercing the thickness direction of the core in which the front and back sheets are adhered along the holes in a non-removable manner so that the front sheet forms ditches extending along the above holes (see Reference 2). Furthermore, as another example, there is known a disposable diaper comprising an absorbent article composed of a liquid pervious front sheet, a liquid impervious back sheet, and an absorbent core which intervenes therebetween in which the absorbent core is covered with an absorbent diffusible sheet in such a manner that at least one ditch is provided on the front sheet side of the absorbent core in the direction from the front sheet to the back sheet so that a bottom part and a side wall part of the ditch are covered with the front sheet in which the absorbent core comprises absorbent fibers and super absorbent polymer particles, said absorbent fibers and super absorbent polymer particles intervene between the front sheet and the back sheet on the bottom part of the ditch (see Reference 3).
Reference 1: Utility Model application unexamined publication No. H1-14707
Reference 2: Patent application unexamined publication No. H9-51913
Reference 3: Patent application unexamined publication No. 2002-165834

In a conventional absorbent article described above, there is an advantage in that a hole or a ditch functions as a guide for liquid discharged on a front surface sheet; however, since an absorbent core adjacent to a hole or a ditch is pressured with a front surface sheet, it causes a disadvantage in that inherent liquid absorbency of an absorbent core is decreased in proximity to a hole or a ditch.

That is, in a conventional absorbent article, a hole or a ditch is generally formed in such a manner that a front surface sheet and a back surface sheet are opposed so as to have a flat absorbent core therebetween in which the front sheet sinks into the back sheet side to adhere thereto at each place that a hole or ditch is provided. Thereby, a flat absorbent core adjacent to a hole or a ditch through a front surface sheet is under the pressure by the front surface sheet, which causes inconvenience in which the inherent absorbent capability of the absorbent core is impaired.

Then, the purpose of this invention is to provide a new absorbent article, having a hole or a ditch that functions as a guide for the liquid discharged on a front surface sheet, and eliminating the above disadvantage of a conventional absorbent article comprising a hole or a ditch.

### SUMMARY OF THE INVENTION

An absorbent article of the present invention comprises a rectangular or nearly rectangular shaped flat absorbent core interposed between a liquid pervious front sheet and a liquid impervious back sheet in which the flat absorbent core interposed between said two sheets is divided into not less than two parts by at least one ditch traversing a point within a circle with a radius of 25 mm from its center to the periphery of the flat absorbent core, in which the ditch has the same depth as the thickness of the flat absorbent core, and is 2 - 6 mm in width while the ditch can be neither directly seen from the front sheet side nor the back sheet side.

A hole or a ditch provided at a conventional absorbent article can be generally seen from a front sheet side. On the other hand, as a hole or a ditch provided at an absorbent core of an absorbent article of the present invention can be directly seen neither from a front sheet side nor a back sheet side, such a ditch will be called a hidden ditch in this specification. Needless to say, both sidewalls of this hidden ditch are composed of longitudinal section of a flat absorbent core, and the top and the bottom of the ditch are closed with the front sheet and a back sheet.

### BRIEF DESCRIPTION OF THE DRAWINGS

FIG. 1 is a plan view showing an example of an absorbent article in which a flat absorbent core is divided into two parts by a hidden ditch extending in the longitudinal direction and imposed between two sheets.
FIG. 2 is a sectional view taken off along the line A-A of FIG. 1.
FIG. 3 is a plan view showing an example of an absorbent article in which a flat absorbent core is divided into three parts by two hidden ditches extending in the longitudinal direction and imposed between two sheets.
FIG. 4 is a sectional view taken off along the line A-A of FIG. 3.
FIG. 5 is a plan view showing an example of an absorbent article in which a flat absorbent core is divided into four parts by three hidden ditches extending in the longitudinal direction and imposed between two sheets.
FIG. 6 is a sectional view taken off along the line A-A of FIG. 5.
FIG. 7 is a plan view showing another example of an absorbent article in which a flat absorbent core is divided by a plurality of hidden ditches extending radially and imposed between two sheets.
FIG. 8 is a plan view of a modified example of the absorbent article shown in FIG.7
FIG. 9 is a vertical sectional view of a liquid injection pipe used in a circular diffusion experiment of saline solution.

### DESCRIPTION OF THE PREFERRED EMBODOMENTS

In this specification, disposable diapers and sanitary napkins are collectively called absorbent articles. However, there are many kinds of absorbent articles, such as with embossing, or those cuffs and/or gathers with elastic materials are provided in order to improve the fitting properties and leakage prevention. In addition, various suggestions are made, for example, about closures provided on disposable diapers, such as its form or positioning. However, such an absorbent article is basically composed of a rectangular or nearly rectangular shaped flat absorbent core interposed between a liquid pervious front sheet and a liquid impervious back sheet regardless of the kind of the article.

In this specification, a nearly rectangular shaped flat absorbent core indicates a flat absorbent core wherein four corners and/or crotch portions (coxitic part) of a rectangular shaped flat absorbent core are trimmed. Therefore, such a nearly rectangular shaped flat absorbent core includes an oval shaped absorbent core and an absorbent core whose plane form is guitar-shaped or hourglass-shaped.

Any liquid pervious sheet, liquid impervious sheet, or absorbent core used in manufacturing conventional absorbent articles can be employed for a front surface sheet, a back surface sheet, or an absorbent core of an absorbent article according to the present invention.. Namely, an absorbent core is generally a laminated body or a flat shaped form composed of, for example, fluffed wooden pulp, super absorbent polymer, synthetic fibers, or the like. Most of a flat absorbent core interposed between two sheets generally has the same thickness. However, the thickness of a central portion can be thicker than that of a surrounding portion.

The most distinctive feature of the absorbent article of the present invention is that a rectangular or nearly rectangular shaped flat absorbent core interposed between a front surface sheet and a back surface sheet is divided into not less than two parts by at least one ditch traversing one point within a circle with a radius of 25 mm from a center of its plane (hereinafter so called as "a center circle") to the periphery of the flat absorbent core in which the ditch is 2 - 6 mm in width while the ditch is a hidden ditch which can be directly seen neither from the front sheet side nor the back sheet side.

In a case that a ditch dividing a flat absorbent core into not less than two parts extends in two directions from one point within the aforementioned center circle, the two directions are typically in an opposite direction of each other. However, the directions do not need to be accurately opposite. In other words, each ditch dividing a flat absorbent core in the thickness direction may, for instance, bend within the center circle. Therefore, a flat absorbent core of the invention can be divided into a plurality of parts by more than one hidden ditch extending radially from one point within the center circle to the periphery of the absorbent core.

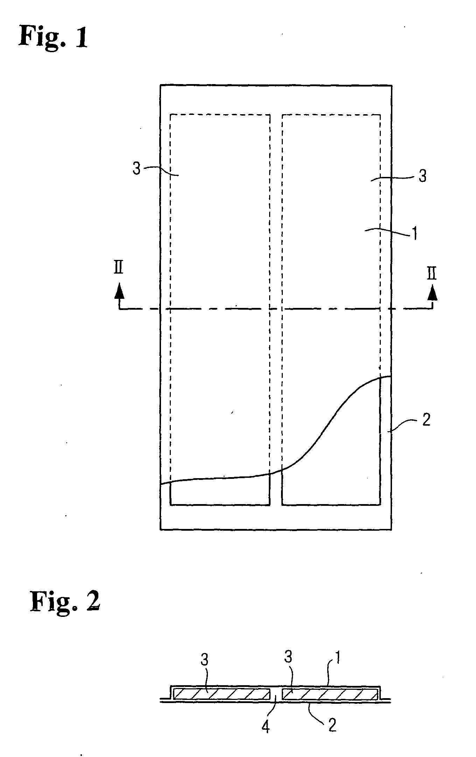

FIGs. 1 - 8 depict absorbent articles of the invention, wherein a flat absorbent core is divided or partitioned into a plurality of parts by one or more than two hidden ditches in which the absorbent core is interposed between a liquid pervious front sheet and a liquid impervious back sheet. In the illustrated absorbent articles, a rectangular shaped absorbent core is utilized as a flat absorbent core before being divided or partitioned into parts by hidden ditch(es). However, as explained previously, a nearly rectangular shaped absorbent core of which four corners and/or crotch portions are trimmed can be replaced with the above absorbent core. When a crotch portion of an absorbent core used in an absorbent article is trimmed so as to deform its plane figure into a hourglass shape or a guitar-shape, crotch portions of a front sheet and a back sheet interposing the core therebetween may be trimmed as well.

FIGs. 1 - 8 show examples in which the plane form of both front sheet and back sheet are also rectangle similar to that of a flat absorbent core for convenience. Thereby, it does not mean that a front surface sheet and a back surface sheet of an actual absorbent article are actually rectangle. Incidentally, in disposable diapers, in case that a rectangular or nearly rectangular shaped flat absorbent core is used, the portions of a front sheet and a back sheet which surround the waist and the stomach when applied to the human body are generally extended in a wing shape. Therefore, in case of applying the present absorbent article to a disposable diaper, such a wing-shaped extension is provided on both front sheet and back sheet interposing the flat absorbent core therebetween.

In addition, although it is not illustrated, according to the invention, it is possible to apply embossing finish on an absorbent article to improve fitting properties and leakage prevention when applied to the human body. Cuffs and/or gathers composed of elastic materials can be provided as well. Further, in a disposable diaper, various types of closures can be provided for fixing around the waist.

In the drawings, reference numeral 1 denotes a liquid pervious inner sheet, and reference numeral 2 denotes a liquid impervious outer sheet, while reference numeral 3 denotes a flat absorbent core which is divided by a hidden ditch 4 in the thickness direction.

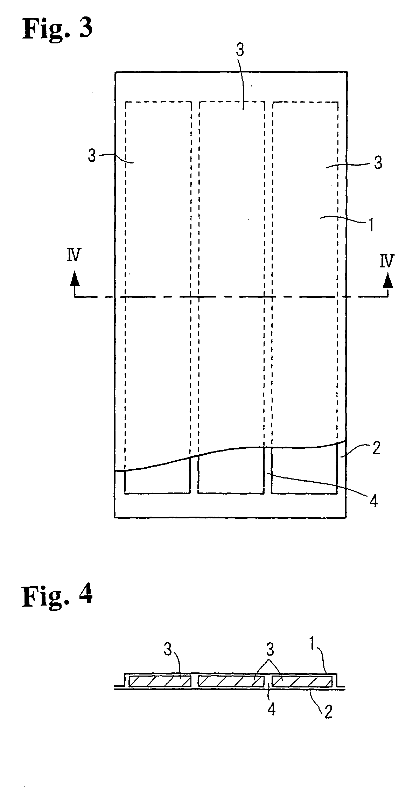

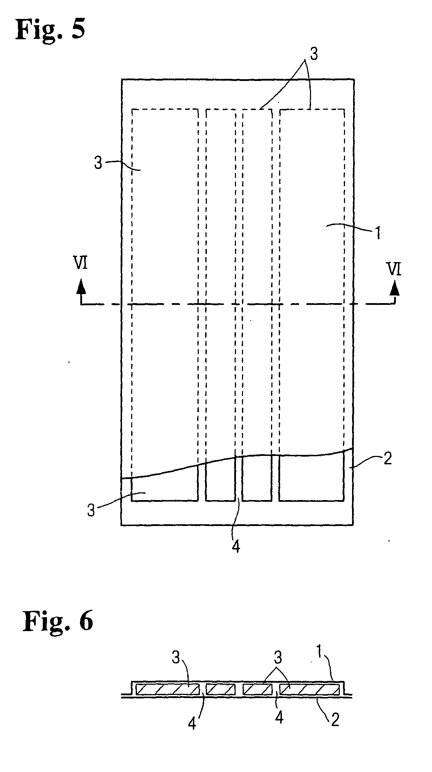

In an absorbent article shown in FIG. 1, a rectangular shaped flat absorbent core is divided by a ditch extending in the longitudinal direction at the center and interposed between two sheets. FIG.3 shows the absorbent article, wherein a rectangular shaped flat absorbent core is divided by two ditches arranged parallel to the center line extending in the longitudinal direction so as to interpose it therebetween. FIG.5 denotes an absorbent article wherein a rectangular shaped flat absorbent core is divided into four parts by three ditches extending in the longitudinal direction at the center.

In any absorbent article, it is preferable for a ditch extending in the longitudinal direction to traverse a circle with a radius of 25 mm (a center circle) whose center is a center of the flat absorbent core, but it is not necessary for each ditch to be straight. For instance, in the absorbent core shown in FIG.5, as all three ditches extending in the longitudinal direction have to traverse the center circle, the ditch arranged at the center is typically straight while other two ditches arranged on both sides of the center one can have greater distance to each other as they leave from the center circle. In other words, each of the two ditches arranged on both sides of the ditch arranged at the center may bend within the center circle.

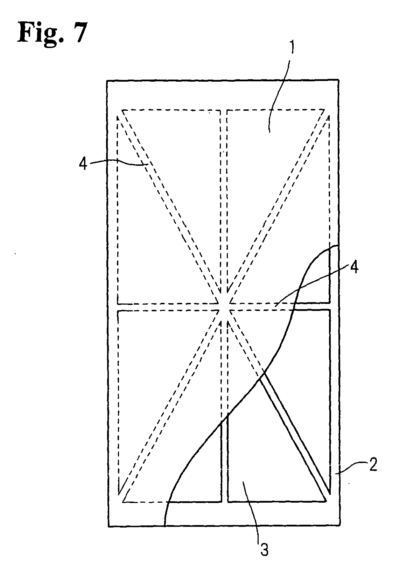

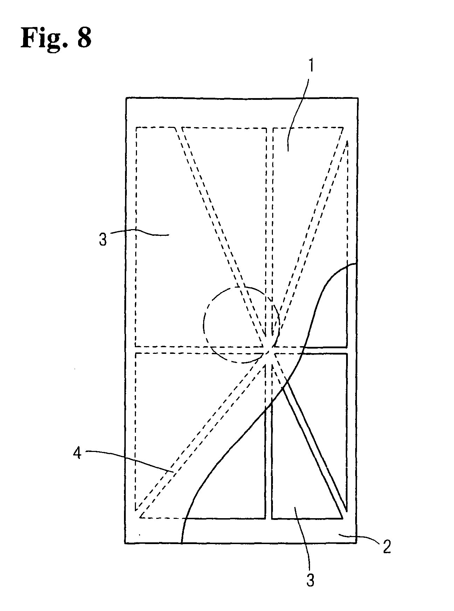

FIGs. 7 and 8 denote an absorbent article, wherein a rectangular shaped flat absorbent core is divided into a plurality of parts by more than one hidden ditch extending radially from one point within a center circle to the periphery of a flat absorbent core. In an absorbent article shown in FIG. 7, a starting point of the ditch extending radially is at the center of the rectangular shaped absorbent core. However, in FIG. 8, a starting point of the ditch extending radially is not at the center of the rectangular shaped flat absorbent core but is within the center circle. A circle depicted with a dotted line indicates the aforementioned center circle with a radius of 25 mm.

In each of the illustrated absorbent articles, a hidden ditch 4 comprises sidewalls composed of sections of a flat absorbent core in which a liquid pervious front sheet and a liquid impervious back sheet are arranged at the top and the bottom of the hidden ditch. Accordingly, the hidden ditch 4 is hollow, and cannot be seen directly from either the front sheet side or back sheet side.

### EXAMPLES

We will explain the present invention more concretely by showing some examples as follows; however, the examples are not intended to limit the present invention.

### Example 1

With an air laid equipment, five layered absorbent core whose weight percentage of NBSK fiber / thermally bonded fiber / super absorbent polymer is 47.1 / 4.3 / 48.6 is prepared.

| | | |
|---|---|---|
| 1^{St} Layer: | NBKP fiber 55g/m² + Thermally bonded fiber 5g/m² | = 60g/m² |
| 2^{nd} Layer: | Super absorbent polymer 85g/m² | = 85g/m² |
| 3^{rd} Layer: | NBKP fiber 55g/m² + Thermally bonded fiber 5g/m² | = 60g/m² |
| 4^{th} Layer: | Super absorbent polymer 85g/m² | =85g/m² |
| 5^{th} Layer: | NBKP fiber 55g/m² + Thermally bonded fiber 5g/m² | = 60g/m² |
| Total: 350g/m² | | |

A 36cm X 15cm flat absorbent core was cut off from the thus obtained absorbent core sheet, divided into two parts by a 6 mm wide ditch along the center line in the longitudinal direction so as to apply 20g/m² basis weight Asahikasei Erutasu Aqua onto a front sheet and a 25 µ thick PE film onto a back sheet to obtain an absorbent core shown in FIG. 1.

### Example 2

The same procedure of Example 1 was repeated to obtain an absorbent core shown in FIG. 1 except for changing the width of the ditch to 4 mm.

### Example 3

The same procedure of Example 1 was repeated to obtain an absorbent core shown in FIG. 1 except for changing the width of the ditch to 2 mm.

### Example 4

An absorbent article shown in FIG. 3 was obtained by using the same size flat absorbent core and the same front sheet and back sheet as Example 1. In this article, each of two ditches was 6 mm wide, and a belt like absorbent core interposed between those ditches was 25 mm wide.

### Example 5

The same procedure of Example 4 was repeated to obtain an absorbent core shown in FIG. 3 except for changing the width of the ditches to 4 mm.

### Example 6

The same procedure of Example 4 was repeated to obtain an absorbent core shown in FIG. 3 except for changing the width of the ditches to 2 mm. Example 7

An absorbent article shown in FIG. 5 was obtained by using the same size flat absorbent core and the same front sheet and back sheet as Example 1. In this article, each of three ditches was 6 mm wide wherein one of those ditches was arranged along the center line of the flat absorbent core in the longitudinal direction, and the width of two belt like absorbent cores interposing the center ditch therebetween was 17 mm, respectively.

### Example 8

The same procedure of Example 7 was repeated to obtain an absorbent core shown in FIG. 5 except for changing the width of the ditches to 4 mm.

### Example 9

The same procedure of Example 7 was repeated to obtain an absorbent core shown in FIG. 5 except for changing the width of the ditches to 2 mm.

### Example 10

An absorbent article shown in FIG. 7 was obtained by using the same size flat absorbent core and the same front sheet and back sheet as Example 1. In this article, the flat absorbent core was divided into eight parts by ditches of 6 mm in width extending radically from the center of the flat absorbent core so as to be interposed between a front sheet and a back sheet.

### Example 11

The same procedure of Example 10 was repeated to obtain an absorbent core shown in FIG. 7 except for changing the width of the ditches extending radically from the center to 4 mm.

### Example 12

The same procedure of Example 10 was repeated to obtain an absorbent core shown in FIG. 7 except for changing the width of the ditches extending radically from the center to 2 mm. A liquid absorption rate, a wet back volume, and a diffusion area ratio were measured about each absorbent article obtained in examples 1 to 12 according to the following method:

The results are shown in Table 1. A comparison example in table 1 reveals the result of an absorbent article obtained by repeating Example 1 except for not dividing the flat absorbent core with a ditch.

### Liquid Absorption Rate:

An injection pipe having an internal diameter of 45 mm was vertically connected to the central portion of an acrylic board of 10 cm X 10 cm X 10 mm in thickness, and a measuring apparatus was prepared in which an opening of the same inner diameter as the injection pipe was provided on the connecting part of the acrylic board. The measuring instrument was gently placed approximately on the center of a front sheet of an absorbent article that was spread on a plane surface. In this instance, the acrylic board is in contact with the front sheet and maintains the status in which the injection pipe stands up vertically. Then, 200 ml of physiological saline of 0.9 weight percentage was poured into the injection pipe from the top so as to keep a liquid level of about 50 mm and the time (second) for absorbing all the physiological saline was measured. Three measuring samples were prepared for each absorbent article, and time for being absorbed and average thereof was evaluated per sample.

### Wet Back Amount:

After each sample used for measuring a liquid absorbing rate, i.e., the samples having absorbed 200ml of physiological saline was left for 10 minutes, 30 sheets of filter papers of 100 mm X 100 mm (trade name:Advantech No.1140, manufactured by Toyo Filter Paper Inc.) were piled onto the central portion of the sample, and left for 5 minutes while loading 10 kg of weight thereon.. Then, an increase of weight of the filter papers was measured as the web back amount.

### Diffusion Area Ratio

A small amount of blue dye was added into 200ml of physiological saline used for measuring a liquid absorption rate as explained above. After the samples absorbed the colored saline, the diffusion area of the colored saline for each sample of the flat absorbent core was measured so as to evaluate the ratio of the diffusion area against the whole area of the flat absorbent core (36 mm in length by 15 mm in width).

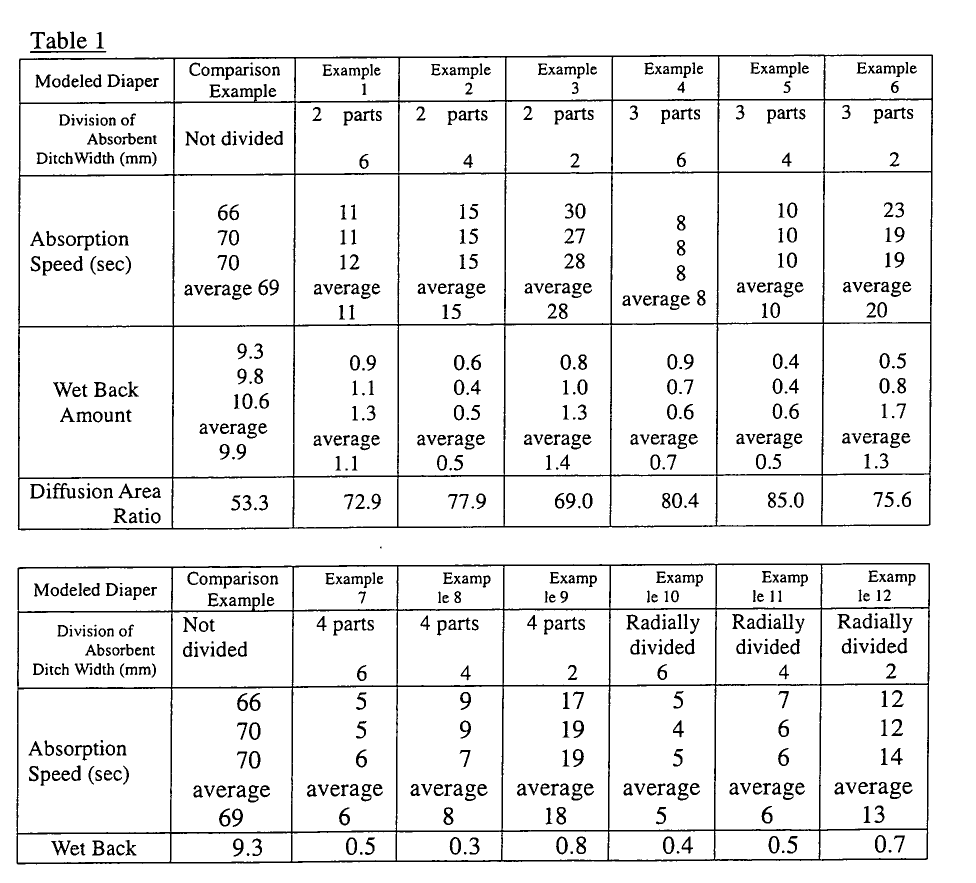

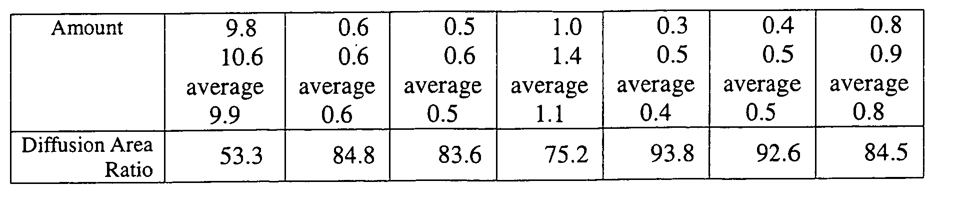

It will be appreciated that each absorbent article according to Examples 1 to 12 has been greatly improved not only in liquid absorption rate but also wet back amount compared with those of the comparison example wherein an absorbent core is not divided by a hidden ditch.

The following experiments were carried out to figure out most effective position for such a hidden ditch to be provided on a flat absorbent core.

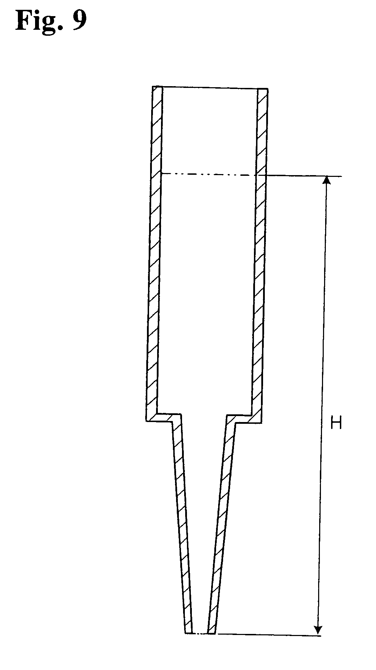

An absorbent article was prepared by repeating the same procedure of Example 1 using an identical flat absorbent core, inner surface, and outer surface except for not being divided by a ditch (same as a comparison example 1 of table 1). A liquid injection pipe shown in FIG. 9 was vertically positioned above the center of the absorbent article spread on a plane surface and 200 ml of physiological saline having concentration of 0.9 weight percentage was poured onto the absorbent article while changing the vertical distance from the outlet of the injection pipe to the absorbent article at a flow rate of 14 ml/sec so as to measure the time (second) for the saline to spread from the place it dropped to a concentric circle having a predetermined radius.

An internal diameter D of a cylindrical tube composing the upper half of the liquid injection pipe was 48-mm while a conical tube composing the lower half was designed to have 100 mm in length, 10 mm of upper internal diameter, and 3 mm of internal diameter of the liquid outlet. Furthermore, in order to maintain the average liquid flow of 14 ml/sec during the experiments, head H of saline in the injection pipe was set at 215 mm before the liquid began to flow.

Table 2 shows the experimental result in which the time for the saline to spread from the place it dropped to a concentric circle having a predetermined radius was measured as to the drop of 5 mm and of 25 mm. According to the experimental result shown in Table 2, the delay of diffusion time was approximately 1.5 second even when the horizontal distance from the drop position to the plane center of the absorbent core was 25 mm, which is within a tolerance of an absorbent article. In addition, a liquid flow free surface of approximately 50 mm in diameter in which its center was the drop position was observed on the surface of the absorbent core when the liquid flowed out. Accordingly, it is apparent from the experimental results that liquid absorbency can be greatly improved by locating a hidden ditch for dividing an absorbent core within a circle having a radius of 25 mm from a plane center of an absorbent core when an outlet for liquid is positioned at the plane center of the absorbent core.

**Table 2**

| Radius of Circular Diffusion Circle of Liquid | Horizontal Distance from Liquid Outlet to Surface of Absorbent Article | |
|---|---|---|
| | Diffusion Time with Distance of 5 mm | Diffusion Time with Distance of 25 mm |
| 10 mm | 0.7 second | 0.8 second |
| 20 mm | 1.2 second | 1.0 second |
| 30 mm | 1.8 second | 1.4 second |
| 40 mm | 2.7 second | 2.1 second |
| 50 mm | 3.0 second | 3.3 second |

In an absorbent article of the invention, since a ditch (hidden ditch) dividing a flat absorbent core into a plurality of parts functions as a guide for liquid discharged onto the absorbent article, the liquid discharged approximately to the center of the absorbent article can be quickly guided to the periphery. Moreover, in the absorbent core, an inner sheet is not pressed even a section adjacent to a ditch which is commonly seen in conventional articles, which allows the absorbent core to perform its essential absorbent capability.

According to the present invention, liquid discharged to the surface can diffusely osmose into the whole absorbent core with little wet back amount, which improves the absorbent efficiency of a base material comprised in an absorbent core. As a result, such a base material for absorbing the same amount of body fluid can be reduced so that an absorbent article such as a resource saving or waste saving type disposable diaper or sanitary napkin can be provide.

## Claims

1. An absorbent article comprising a rectangular or a nearly rectangular shaped flat absorbent core interposed between a liquid pervious front sheet and a liquid impervious back sheet, wherein the flat absorbent core interposed between said two sheets is divided into not less than two parts by at least one ditch traversing a point within a circle with a radius of 25 mm from its center to the periphery of the flat absorbent core, wherein the ditch is hollow and 2 - 6 mm in width, and has the same depth as the thickness of the flat absorbent core while having.

2. The absorbent article defined in claim 1, wherein the flat absorbent core is divided into two parts by said one ditch extending in the longitudinal direction.

3. The absorbent article defined in claim 1, wherein the flat absorbent core is divided into three parts by said two ditches extending in the longitudinal direction.

4. The absorbent article defined in claim 1, wherein the flat absorbent core is divided into a plurality of parts by at least one ditch extending radically from a point within a circle with a radius of 25 mm from the center of the absorbent core to the periphery of said core.
